# EUROPÄISCHE PATENTSCHRIFT

(11) **EP 0 815 839 B1**
(45) Veröffentlichungstag und Bekanntmachung des Hinweises auf die Patenterteilung: **06.10.2004**
(21) Anmeldenummer: 97111330.3
(22) Anmeldetag: 04.07.1997
(51) Int. Cl.: A61K 7/48

(54) **Kosmetische oder pharmazeutische Mittel auf Basis von Acrylat Polymeren**
Cosmetic or pharmaceutical composition containing an acrylic polymer
Composition cosmétique ou pharmaceutique contenant un polymère acrylique

(30) Priorität: 05.07.1996 DE 19627204
(43) Veröffentlichungstag der Anmeldung: 07.01.1998
(73) Patentinhaber: BASF AKTIENGESELLSCHAFT, 67056 Ludwigshafen (DE)
(72) Erfinder: Schehlmann, Volker, Dr., 67354 Römerberg (DE); Schade, Christian, Dr., 67061 Ludwigshafen (DE); Sanner, Axel, Dr., 67227 Frankenthal (DE); Sperling, Karin, Dr., 67433 Neustadt (DE); Wekel, Hans-Ulrich, Dr., 67158 Ellerstadt (DE)
(74) Vertreter: Kinzebach, Werner, Dr.

(56) Entgegenhaltungen:
- EP-A- 0 013 836
- EP-A- 0 391 274
- DE-A- 4 342 281
- FR-A- 2 492 658
- US-A- 4 423 031

## Beschreibung

Die vorliegende Erfindung betrifft kosmetische oder pharmazeutische Mittel zur Anwendung auf der Haut, die zur Erhöhung der Wasserfestigkeit Polymerisate oder Copolymerisate enthalten, die in Gegenwart von Kettentibertragungereagenzien durch radikalische Emulsions- oder Suspensionspolymerisation erhältlich sind.

Bei kosmetischen oder pharmazeutischen Mitteln, die auf die Haut aufgetragen werden, ist häufig eine gute Wasserfestigkeit wünschenswert. Derartige Mittel sind insbesondere von Interesse, wenn ein häufiger Kontakt der Haut mit Wasser oder wäßrigen Flüssigkeiten, wie z.B. Schweiß, beobachtet wird, und eine erhöhte Naßabriebfestigkeit zu einer verlängerten Wirksamkeit führt. Um die gewünschte Wasserfestigkeit zu erreichen, werden solchen Mitteln häufig Polymere zugesetzt. Weiterhin ist zu beachten, daß diese Mittel die Haut weder austrocknen noch zu Entzündungen führen, sondern idealerweise feuchtigkeitspendende Wirkung besitzen und sich gleichmäßig auf der Haut verteilen lassen sollen.

Die DE-2 833 711 beschreibt Sonnenschutzformulierungen, die zur Erhöhung der Wasserfestigkeit öllösliche Acrylatpolymere enthalten. Es werden sowohl aus Acrylsäureestern hergestellte Homopolymere beschrieben als auch Copolymere aus Acrylsäureestern und carboxylgruppenhaltigen Monomeren, wie (Meth)acrylsäure oder Itaconsäure. Die Polymere werden ohne Kettenübertragungsreagenzien und insbesondere durch Lösungspolymerisation in einem kosmetisch brauchbaren Öl, wie Isopropylpalmitat hergestellt und direkt in Form der erhaltenen Lösung zu einem Sonnenschutzmittel formuliert.

Copolymere aus N-Vinylpyrrolidon und α-Olefinen und deren Anwendung in kosmetischen Formulierungen werden beispielsweise in der US 5 219 559 und der US 3 423 381 beschrieben. Sie werden durch Lösungspolymerisation ohne Verwendung eines Kettenübertragungsmittels und nach Entfernen des Lösungsmittels weiterverarbeitet.

Diese aus dem Stand der Technik bekannten Mittel weisen zwar eine erhöhte Wasserfestigkeit auf, haben aber verschiedene Nachteile, die insbesondere auf das dort verwendete Verfahren zur Herstellung der beschriebenen Polymere zurückzuführen sind. Beispielsweise wird häufig ein hoher Gehalt an Restmonomeren und anderen geruchsbildenden Verunreinigungen beobachtet, die aus toxikologischen und olfaktorischen Gründen in pharmazeutischen und kosmetischen Mitteln zu vermeiden sind. Die notwendige Entfernung dieser niedermolekularen Bestandteile ist oft nur mit großem technischem Aufwand zu bewerkstelligen oder, wie bei einer Lösungspolymerisation, sogar technisch in der Praxis unmöglich.

Ein weiterer Nachteil dieser Produkte ist die vergleichsweise schlechte Handbarkeit. Bei einer Lösungspolymerisation, insbesondere von Acrylestern, fällt das Polymer als ölige bis wachsartige Masse an, woraus Einschränkungen bei der Wahl der anschließenden pharmazeutischen oder kosmetischen Formulierung resultieren und für den Hersteller derartiger Mittel ein relativ großer technischer Aufwand bei der Verarbeitung entsteht.

Der vorliegenden Erfindung liegt daher die Aufgabe zugrunde, kosmetische oder pharmazeutische Mittel auf Acrylatbasis mit hoher Wasserfestigkeit zur Verfügung zu stellen, wobei die zu diesem Zweck enthaltenen Polymere farblos, geruchsarm und gut handhabbar sein sollen.

Überraschenderweise wurde nun gefunden, daß sich solche (Meth)acrylsäureesterpolymerisate oder deren Copolymerisate mit radikalisch copolymerisierbaren Monomeren zur Lösung dieser Aufgabe eignen, die durch radikalische Emulsions- oder Suspensionspolymerisation in Gegenwart von Kettenübertragungsreagenzien erhältlich sind.

Gegenstand der vorliegenden Erfindung ist also ein kosmetisches oder pharmazeutisches Mittel, das wenigstens ein Polymerisat oder Copolymerisat enthält, welches durch radikalische Emulsions- oder Suspensionspolymerisation in Gegenwart von wenigstens einem Kettenübertragungsreagens erhältlich ist, eine Glasübergangstemperatur mehr als -35°C (bestimmt mittels DSC) und einen Gehalt an organischen flüchtigen Bestandteilen ≤ 0,5 Gew.-% aufweist und zu mindestens 20 Gew.-% aus einem (Meth)acrylsäureester aufgebaut ist.

Insbesondere geeignet sind Mittel, die wenigstens ein Polymerisat oder Copolymerisat enthalten, welches aus
a) 40 bis 100 Gew.-% wenigstens eines C₁-C₃₀-(Meth)acrylsäureesters als Monomer A,
b) 0 bis 30 Gew.-% wenigstens eines radikalisch copolymerisierbaren wasserlöslichen Monomers als Monomer B,
c) 0 bis 40 Gew.-% wenigstens eines radikalisch copolymerisierbaren gegebenenfalls N-C₁-C₁₈-Alkyl- oder -Hydroxyalkylsubstituierten (Meth)acrylamids als Monomer C und
d) 0 bis 30 Gew.-% wenigstens eines radikalisch copolymerisierbaren Monomers als Monomer D
aufgebaut ist. Wenn die Monomere B, C und D vorhanden sind, sind sie vorzugsweise zu mindestens 1 Gew.-% enthalten.

Bei den zum Aufbau der erfindungsgemäßen Polymere verwendeten (Meth)acrylsäureestern (Monomere A) handelt es sich vorzugsweise um Ester der (Meth)acrylsäure mit C₁-C₃₀-Alkylalkoholen, beispielsweise Methylmethacrylat, Ethylacrylat, Ethylmethacrylat, n-Propylacrylat, n-Propylmethacrylat, iso-Propylacrylat, iso-Propylmethacrylat, n-Butylacrylat, n-Butylmethacrylat, iso-Butylacrylat, iso-Butylmethacrylat, t-Butylacrylat, t-Butylmethacrylat, Pentylacrylat, Pentylmethacrylat, n-Hexylacrylat, n-Hexylmethacrylat, n-Heptylacrylat, n-Heptylmethacrylat, n-Octylacrylat, n-Octylmethacrylat, 2-Ethylhexylacrylat, 2-Ethylhexylmethacrylat, Decylacrylat, Decylmethacrylat, Laurylacrylat, Laurylmethacrylat, Palmitylacrylat, Palmitylmethacrylat, Stearylacrylat, Stearylmethacrylat, Hydrenol(meth)acrylat, Behenyl(meth)-acrylat, Polyisobuten(meth)acrylat, Phenoxyethylacrylat oder Phenoxyethylmethacrylat, oder um C₅-C₆-Cycloalkyl(meth)acrylate, wobei der Cycloalkylrest durch 1, 2 oder 3 C₁-C₄-Alkylgruppen substituiert sein kann, beispielsweise Cyclohexylacrylat, Cyclohexylmethacrylat, 4-Methylcyclohexylacrylat oder 4-Methylcyclohexylmethacrylat.

Bevorzugt werden C₂-C₆-(Meth)acrylsäureester und C₁₂-C₂₂-(Meth) acrylsäureester.

Insbesondere bevorzugt als Monomer A sind t-Butylacrylat, t-Butylmethacrylat, iso-Butylacrylat, iso-Butylmethacrylat und t-Butylacrylat oder t-Butylmethacrylat.

Zum Aufbau der erfindungsgemäßen Polymere können auch radikalisch polymerisierbare wasserlösliche Monomere (Monomere B) herangezogen werden, die die physikalisch-chemischen Eigenschaften, wie die Glasübergangstemperatur und Löslichkeit des resultierenden Copolymerisats sowie die Substantivität eines Mittels, welches diese Polymere enthält, auf der Haut beeinflussen. Auch können sie die Verträglichkeit mit anderen Komponenten der kosmetischen oder pharmazeutischen Zubereitung verbessern. Weiterhin kann die Verwendung dieser Monomere B zum Aufbau der erfindungsgemäßen Copolymerisate der Optimierung des sogenannten Hautgefühls des Mittels nach der Anwendung dienen.

Bevorzugt sind deshalb kosmetische oder pharmazeutische Mittel, die Copolymerisate enthalten, welche aus wenigstens einem Monomer A und aus wenigstens einem Monomer B aufgebaut sind.

Erfindungsgemäß geeignete Monomere B finden sich unter den monoethylenisch ungesättigten C₃-C₅-Carbonsäuren, wie Acrylsäure, Methacrylsäure, Crotonsäure, Itaconsäure, Maleinsäure, Fumarsäure, N-Vinyl-substituierte Lactame, z.B. N-Vinylpyrrolidon, N-Vinylvalerolactam und N-Vinylcaprolactam, und hydroxylgruppenhaltigen C₂-C₆-(Meth)acrylsäureester, wie Hydroxyethyl(meth)acrylat.

Die Eigenschaften der so erhaltenen, aus den Monomeren A und B aufgebauten Copolymerisate können noch weiter verbessert werden, indem man je nach Verwendungszweck ein weiteres Monomer (Monomer C) einpolymerisiert, welches das Monomer B ganz oder teilweise ersetzen kann.

Als Monomer C eignen sich vorzugsweise gegebenenfalls N-C₁-C₁₈-Alkyl- oder -Hydroxyalkylsubstituierte (Meth)acrylamide, wie Acrylamid, Methacrylamid, N-Methylacrylamid, N-Methylmethacrylamid, N-Ethylacrylamid, N-Ethylmethacrylamid, N-Propylacrylamid, N-Propylmethacrylamid, N-iso-Propylacrylamid, N-iso-Propylmethacrylamid, N-n-Butylacrylamid, N-n-Butylmethacrylamid, N-iso-Butylacrylamid, N-iso-Butylmethacrylamid, N-t-Butylacrylamid, N-t-Butylmethacrylamid, N-Pentylmethacrylamid, N-Hexylacrylamid, N-Hexylmethacrylamid, N-Heptylacrylamid, N-Heptylmethacrylamid, N-Octylacrylamid, N-Octylmethacrylamid, N-2-Ethylhexylacrylamid, N-2-Ethylhexylmethacrylamid, N-Nonylacrylamid, N-Nonylmethacrylamid, N-Decylacrylamid, N-Decylmethacrylamid, N-Laurylacrylamid, N-Laurylmethacrylamid, N-Palmitylacrylamid, N-Palmitylmethacrylamid, Stearylacrylamid, N-Stearylmethacrylamid, N-Hydroxyethylacrylamid, N-Hydroxyethylmethacrylamid, N-Hydroxypropylacrylamid und N-Hydroxypropylmethacrylamid. Bevorzugt sind C₄-C₁₈-Alkylsubstituierte (Meth)acrylamide und insbesondere N-t-Butyl(meth)-acrylamid.

Schließlich können erfindungsgemäß geeignete Copolymerisate ein weiteres Monomer (Monomer D) einpolymerisiert enthalten. Dabei können die Monomere D mit den Monomeren A je nach Verwendungszweck alleine oder in Kombination mit den Monomeren B und/oder C zum Aufbau der erfindungsgemäßen Polymere copolymerisiert werden.

Erfindungsgemäß bevorzugte Monomere D sind C₁-C₃₀-Vinylester, wie Vinylacetat, Vinylproprionat, Vinylbutyrat, Vinylvalerat, Vinyl-2-ethylhexanoat, Vinyldecanoat, Vinylpalmitat, Vinylstearat und Vinyllaurat, C₁-C₃₀-Vinylether, wie Methylvinylether, Ethylvinylether, N-Propylvinylether, iso-Propylvinylether, n-Butylvinylether, iso-Butylvinylether, 2-Ethylhexylvinylether, Decylvinylether, Dodecylvinylether, Stearylvinylether, 2-(Diethylamino)ethylvinylether, 2-(Di-n-Butylamino)ethylvinylether und Methyldiglykolvinylether, vinylaromatische Verbindungen, z.B. styrol und substituierte Styrole, wie p-Methylstyrol und α-Methylstyrol, und Si-haltige Monomere, insbesondere ungesättigte Polysiloxane.

Bevorzugte Monomere D sind Vinylester, insbesondere C₁₂-C₂₂-Vinylester, C₁₂-C₂₂-Vinylether, Styrol und ungesättigte Polysiloxane der Formel I: 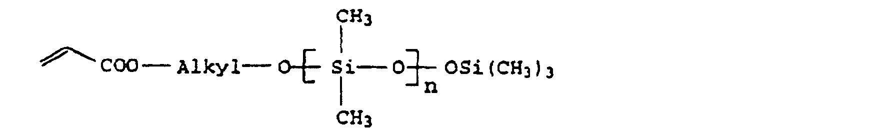 worin n = 6-50 und Alkyl für einen geradkettigen oder verzweigten C₂-C₁₈-Alkylenrest steht.

Die physikalisch-chemischen Eigenschaften der erfindungsgemäßen Polymere oder Copolymere hängen von der Art der verwendeten Monomere, deren relativen Mengenverhältnissen und den Polymerisationsbedingungen ab und lassen sich durch die Wahl geeigneter Parameter gezielt beeinflussen.

Die erfindungsgemäßen Polymerisate besitzen eine Glasübergangstemperatur Tg von mehr als -35°C. Vorzugsweise beträgt die Glasübergangstemperatur ≥ 0°C und insbesondere bevorzugt sind Polymerisate oder Copolymerisate, deren Glasübergangstemperatur oberhalb von 35°C liegt. Erfindungsgemäße Polymerisate mit niedrigen Glasübergangstemperaturen werden bevorzugt durch Emulsionspolymerisation hergestellt, während Polymere mit höheren Glasübergangstemperaturen vorzugsweise durch Suspensionspolymerisation erhältlich sind. Die Obergrenze der Glasübergangstemperatur beträgt im allgemeinen 120°C. Ganz besonders bevorzugte Polymerisate oder Copolymerisate besitzen eine Glasübergangstemperatur im Bereich von 35°C bis 100°C.

Die Polymerisate besitzen im allgemeinen K-Werte (nach H. Fikkentscher, Cellulose-Chemie 13 (1932), Seiten 58 bis 64 und 71 und 74) von > 15, vorzugsweise > 20. Insbesondere bevorzugt sind Polymere, deren K-Wert im Bereich von 20 bis 60 liegt.

Die Polymerisation der oben beschriebenen Monomere erfolgt durch Emulsions- oder Suspensionspolymerisation in einem wäßrigen Medium, vorzugsweise in Wasser. Unter wäßrigem Medium sind hier auch Mischungen aus Wasser und mit Wasser mischbaren Lösungsmitteln, wie Methanol, Ethanol, Isopropanol etc., zu verstehen.

Die Polymerisation erfolgt üblicherweise unter Sauerstoffausschluß bei Temperaturen von beispielsweise 30 bis 100°C, vorzugsweise 50 bis 95°C. Die Emulsionspolymerisation kann diskontinuierlich oder kontinuierlich durchgeführt werden, während die Suspensionspolymerisation in der Regel diskontinuierlich ausgeführt wird.

Die erfindungsgemäße Polymerisation wird radikalisch durchgeführt. Als Polymerisationsinitiatoren kommen die bei radikalischen Polymerisationen üblicherweise verwendeten Verbindungen in Betracht, die unter den Polymerisationsbedingungen Radikale liefern, z.B. Peroxide, Hydroperoxide, Peroxodisulfat, Percarbonat, Peroxiester, Wasserstoffperoxid und Azoverbindungen. Beispiele für Initiatoren sind Wasserstoffperoxid, Dibenzoylperoxid, Dicyclohexylperoxiddicarbonat, Dilaurylperoxid, Methylethylketonperoxid, Acetylacetonperoxid, t-Butylhydroperoxid, Cumolhydroperoxid, t-Butylperneodecanoat, t-Amylperpivalat, t-Butylperpivalat, t-Butylperneohexanoat, t-Butylper-2-ethylhexanoat, t-Butylperbenzoat, Lithium-, Natrium-, Kalium- und Ammoniumoxodisulfat, Azoisobutyronitril, 2,2'-Azobis(2-amidinopropan)dihydrochlorid, 2-(Carbamoylazo)isobutyronitril und 4,4'-Azobis(4-cyanovaleriansäure). Auch Photoinitiatoren sind brauchbar. Die Initiatoren werden üblicherweise in Mengen bis zu 10, vorzugsweise 0,02 bis 5 Gew.-%, bezogen auf die zu polymerisierenden Monomere eingesetzt.

Die Initiatoren können allein oder in Mischung untereinander verwendet. Auch die Anwendung bekannter Redoxkatalysatoren, bei denen die reduzierende Komponente in der Regel im molaren Unterschuß angewendet wird, sind geeignet. Als Redox-Katalysatoren kommen weiterhin reduzierende Verbindungen, wie Ascorbinsäure, Alkalimetall- oder Ammoniumsulfite, -bisulfite, -thiosulfate, - dithionite und -tetrathionate oder reduzierend wirkende Phosphorverbindungen, in denen Phosphor eine Oxidationszahl von 1 bis 4 hat, wie beispielsweise Natriumhypophosphit, phosphorige Säure und Phosphite, in Betracht.

Um das Molekulargewicht der Polymerisate zu steuern, wird die Polymerisation in Gegenwart von Kettenübertragungsreagenzien durchgeführt. Als Kettenübertragungsreagenzien eigenen sich beispielsweise Aldehyde, wie Acetaldehyd, Propionaldehyd, N-Butyraldehyd und Isobutyraldehyd, Ameisensäure, Ammoniumformiat, Hydroxylammoniumsulfat und Hydroxylammoniumphosphat. Weiterhin können Kettenübertragungsreagenzien eingesetzt werden, die Schwefel in organisch gebundener Form enthalten, wie SH-Gruppen aufweisende organische Verbindungen, wie Thioäpfelsäure, Thioglykolessigsäure, Ethylhexyldiglykolat, Mercaptoessigsäure, Mercaptopropionsäure, Mercaptoethanol, Mercaptopropanol, Mercaptobutanol, Mercaptohexanol, Didecylmercaptan und T-Dodecylmercaptan. Weiterhin können als Regler können weiterhin halogenierte Kohlenwasserstoff verwendet werden, wie Bromtrichlormethan und Tetrachlorkohlenstoff. Bevorzugt werden Ethylhexyldiglykolat und Mercaptoethanol. Die Menge an Kettenübertragungsreagenz beträgt 0,1 bis 10, vorzugsweise 0,1 bis 5 Gew.-%, bezogen auf die Menge an Monomer.

Die Polymerisation kann in Anwesenheit eines für diese Zwecke üblichen Emulgators und/oder Schutzkolloids durchgeführt werden. Geeignete Schutzkolloide sind beispielsweise Polyvinylalkohole, Cellulosederivate oder Polyvinylpyrrolidone. Die Emulgatoren können anionischer, kationischer oder nichtionischer Natur sein. Geeignete Emulgatoren sind z.B. ethoxylierte Mono-, Di- und Trialkylphenole (EO-Grad 3 bis 50, Alkylrest: C₄-C₉), ethoxylierte Fettalkohole (EO-Grad: 3 bis 50, Alkylrest C₈-C₃₆), ethoxylierte Sorbitanester, sowie Alkali- und Ammoniumsalze von Alkylsulfaten (Alkylrest: C₈ bis C₁₂), von Schwefelsäurehalbestern ethoxylierte Alkanole (EO-Grad 4 bis 30, Alkylrest C₁₂ bis C₁₈) und ethoxylierte Alkylphenole (EO-Grad: 3 bis 50, Alkylrest C₄ bis C₉), von Alkylsulfonsäuren (Alkylrest: C₁₂ bis C₁₈), von Ligninsulfonsäure und von Alkylarylsulfonsäuren (Alkylrest: C₉ bis C₁₈). Als besonders geeignete Emulgatoren haben sich die Alkyldiphenyloxysulfonate erwiesen.

Die durch Emulsionspolymerisation gewonnenen Dispersionen lassen sich effektiv durch Einleiten von Wasserdampf oder Stickstoff von den aus toxikologischen und olfaktorischen Gründen unerwünschten Restmonomeren und anderen niedermolekularen Geruchsträgern befreien. Diese Dispersionen können direkt in den entsprechenden kosmetischen oder pharmazeutischen Mitteln, insbesondere Cremes oder Lotionen, eingesetzt werden. Die Emulsionscopolymersate lassen sich außerdem bei hinreichend hoher Glasübergangstemperatur des Polymerisats problemlos mittels Sprüh- oder Gefriertrocknung oder auch durch Koagulation und anschließende Trocknung in sehr gut verarbeitbare und handhabbare Pulver überführen. Diese Verfahrensschritte tragen weiter zur Minimierung des Gehalts an niedermolekularen flüchtigen Bestandteilen bei.

Die Suspensionspolymerisate lassen sich ebenfalls leicht mittels Wasser oder Wasserdampf extrahieren und zeichnen sich außerdem durch eine hervorragende Handhabbarkeit aus. Die frei fließenden Pulver können den Erfordernissen entsprechend zusammen mit den verschiedenen kosmetischen oder pharmazeutischen Hilfsstoffen, insbesondere Ölen formuliert werden.

Die so erhaltenen Emulsions- oder Suspensionspolymerisate zeichnen sich durch einen außerordentlich geringen Gehalt an organischen flüchtigen Bestandteilen aus, der 0,5 Gew.-% nicht übersteigt. Die Polymerisate sind farblos, nahezu geruchslos und weitgehend öllöslich.

Die oben beschriebenen Polymerisate oder Copolymerisate verleihen den erfindungsgemäßen Mitteln erhöhte Naßabriebfestigkeit. Vorzugsweise enthalten die Mittel 0,2 bis 20, insbesondere 0,5 bis 10 Gew.-% an Polymerisat, bezogen auf das Gesamtgewicht des Mittels.

Ein derartiges kosmetisches oder pharmazeutisches Mittel kann beispielsweise als Öl, Emulsion, z.B. Creme, Lotion oder Milch, Gel, Pommade oder Spray vorliegen. Die Zusammensetzung und die Verfahren zur Formulierung dieser verschiedenen Applikationsformen sind dem Fachmann bekannt.

Als Ölgrundlage der erfindungsgemäßen Mittel kommen beispielsweise folgende Öle bzw. deren Mischungen in Frage: Pflanzliche Öle, wie Süßmandelöl, Avocadoöl, Ricinusöl, Olivenöl, Traubenkernöl, Nelkenöl, Kolzaöl, Erdnußöl, Maisöl, Haselnußöl, Jojobaöl, Kartamoöl oder Weizenkeimöl, Kohlenwasserstofföle, wie Paraffinöl, Purzellinöl, Perhydrosqualen und Lösungen von mikrokristallinen Wachsen in diesen Ölen, Mineralöle oder Silikonöle. Man kann auch andere synthetische Produkte, wie beispielsweise Ester verwenden, insbesondere Isopropylpalmitat, Isopropylmyristat, Butylmyristat, Cetylmyristat, Hexadecylstearat, Ethylpalmitat, sowie Triglyceride von Octan-, Decansäuren und Cetylricinoleat.

Auch können die erfindungsgemäßen Mittel bestimmte Wachse enthalten, insbesondere Karnaubawachs, Bienenwachs, Ozokerit oder Kandelillawachs.

Geeignete Emulgatoren zum Aufbau der erfindungsgemäßen Emulsionen sind übliche anionaktive, kationaktive, amphotere und nicht-ionische Emulgatoren.

Weiter können die Mittel übliche Zusatzstoffe umfassen, wie Konservierungsmittel, Antioxidantien, Parfums, Farbstoffe, Pigmente, feuchthaltende Mittel, Füllstoffe, wie Talkum, Nylon-, Seiden-, Stärke- oder Polyethylenpulver, Verdickungsmittel, Stabilisatoren, Puffer, Spreitmittel, Säuren, Basen, insbesondere Amine zur partiellen oder vollständigen Neutralisation von Carboxylgruppen, Sonnenschutzfilter sowie pharmazeutische Wirkstoffe, wie Liporegulatoren, antiinflammatorische Mittel, Antibiotika, keralytische Mittel, Vitamine, adstringierende Mittel, antifungische Mittel, insektenabwehrende Mittel oder die Gefäße zusammenziehende Mittel.

Somit finden die erfindungsgemäßen kosmetischen und pharmazeutischen Mittel beispielsweise in Sonnenschutzzubereitungen, Gesichts-, Körper- oder Handpflegemitteln, Insektenschutzmitteln, Lippenstiften, Make-up's, Lidschatten, Wimperntusche, Babycremes, Zubereitungen zum Schutz der Haut bei Inkontinenz, Schutzcremes gegen den Kontakt mit Chemikalien oder Pestiziden oder sonstigen pharmazeutischen Zubereitungen zum Auftragen auf die Haut Verwendung.

Die nachfolgenden Beispiele erläutern die Erfindung ohne sie zu begrenzen.

Im weiteren gelten die folgenden Abkürzungen:
- EA: Ethylacrylat
- BA: Butylacrylat
- EHA: 2-Ethylhexylacrylat
- LA: Laurylacrylat (C12-C14-Alkohol-Acrylsäureester)
- StA: Stearylacrylat
- StMA: Stearylmethacrylat
- tBA: t-Butylacrylat
- tBMA: t-Butylmethacrylat
- iBMA: iso-Butylmethacrylat
- MMA: Methylmethacrylat
- VP: Vinylpyrollidon
- AS: Acrylsäure
- MAS: Methacrylsäure
- NTBAM: N-t-Butylacrylamid
- HEMA: Hydroxyethylmethacrylat
- AA: Acrylamid
- MAA: Methacrylamid
- BTCM: Bromtrichlormethan
- EHTG: Ethylhexythioglycolat
- ME: Mercaptoethanol
- S: Styrol

### 1. Herstellung erfindungsgemäßer Copolymerisate:

### Verfahren 1: Emulsionspolymerisation

Zu einer Lösung aus 3,9 g eines geeigneten Emulgators oder einer Emulgatormischung in 850 g Wasser gibt man unter Rühren innerhalb von 2 Stunden bei ca. 80°C 450 g der zu polymerisierenden Monomermischung, das Kettenübertragungsreagenz und 1,1 g Initiator. Nach beendetem Zulauf wird ca. 1,5 Stunden bei ca. 80°C weitergerührt, und gegebenenfalls zur Restmonomerenreduzierung durch Zusatz eines Redoxsystems vollständig auspolymerisiert. Anschließend wird Wasserdampf eingeleitet, bis der Anteil an wasserdampfflüchtigen Komponenten auf das angestrebte Niveau abgesenkt ist. Die Dispersion wird dann in bekannter Weise durch Sprühtrocknung oder Gefriertrocknung oder Koagulation in ein Pulver überführt. Nach diesem Verfahren wurden Polymerisate unter Verwendung der in der nachfolgenden Tabelle angegebenen Monomeren und Kettenübertragungsreagenzien hergestellt:

**Tabelle 1**

| Beispiel | Monomere | Monomerverhältnis | Kettenübertragungsreagenz (bez. Monomer) | T_{g} [°C] |
|---|---|---|---|---|
| 1 | tBMA/EA | 90/10 | 0,6 % BTCM | 85 |
| 2 | tBMA/EHA | 80/20 | 1,4 % EHTG | 61 |
| 3 | tBA/EHA | 90/10 | 0,8 % EHTG | 39 |
| 4 | iBMA/BA | 85/15 | 1,0 % BTCM | 41 |
| 5 | tBMA/NTBAM/EA | 80/10/10 | 1,2 % EHTG | 81 |
| 6 | tBMA/MMA/EHA | 80/10/10 | 1,5 % EHTG | 78 |
| 7 | tBMA/EHA/MAS | 80/17/3 | 1,3 % EHTG | 62 |
| 8 | tBMA/EHA/AS | 80/18/2 | 1,2 % EHTG | 62 |
| 9 | tBMA/EHA/HEMA | 80/10/10 | 1,5 % EHTG | 66 |
| 10 | tBMA/EHA/AA | 80/17/3 | 1,3 % EHTG | 67 |
| 11 | tBMA/EHA/MAA | 85/15/5 | 1,0 % EHTG | 68 |
| 12 | tBMA/EHA/MAS | 70/10/20 | 1,0 % EHTG | 81 |
| 13 | tBMA/EA/MAS | 60/20/20 | 0,5 % EHTG | 80 |
| 14 | tBA/EA/MAS | 55/20/25 | 0,8 % EHTG | 65 |
| 15 | tBA/NTBAM/MAS | 40/30/30 | 0,3 % BTCM | 76 |
| 16 | tBA/EHA/VP | 80/17/3 | 0,9 % EHTG | 25 |

### Verfahren 2: Suspensionspolymerisation

Zu einer Lösung aus 0,9 g Polyvinylpyrrolidon, M_{w} ca. (1,0 - 1,5) x 10⁶ und 375 g Wasser gibt man unter Rühren 135 g der zu polymerisierenden Monomermischung und das Kettenübertragungsreagenz. Es wird auf 80°C erhitzt und die erste Teilmenge des Initiators, 0,5 g t-Butylper-2-ethylhexanoat, zugegeben. Es wird ca. 3,5 Stunden bei 80°C weitergerührt, nochmals 0,5 g Initiator zugegeben und 3,5 Stunden gerührt. Zur Entfernung von Restmonomeren wird in die Suspension Dampf eingeleitet bis der Anteil an wasserdampfflüchtigen Komponenten auf das angestrebte Niveau abgesenkt ist. Nach Abkühlen wird das Polymerisat abfiltriert und getrocknet. Nach diesem Verfahren wurden Polymerisate unter Verwendung der in der nachfolgenden Tabelle 2 angegebenen Monomeren und Kettenübertragungsreagenzien hergestellt:

**Tabelle 2**

| Beispiel | Monomere | Monomerverhältnis | Kettenübertragungsreagenz | T_{g} [°C] |
|---|---|---|---|---|
| 17 | tBMA/LA | 90/10 | 1,0 % ME | 80 |
| 18 | tBMA/StA | 70/30 | 1,5 % ME | 72 |
| 19 | tBMA/EHA | 80/20 | 1,2 % ME | 60 |
| 20 | tBMA/HEMA | 95/5 | 1,0 % ME | 102 |
| 21 | tBMA/VP | 95/5 | 0,5 % ME | 103 |
| 22 | iBMA/StMA | 70/30 | 1,5 % ME | 38 |
| 23 | tBA/EHA/AS | 80/18/2 | 1,2 % ME | 30 |
| 24 | tBMA/LA/S | 80/10/10 | 1,0 % ME | 82 |

Alle in den Beispielen angeführten Polymerisate weisen als 10%ige Lösung in THF eine Farbzahl nach Gardner ≤ 2 auf, enthalten weniger als 0,5 % flüchtige organische Bestandteile und sind nahezu geruchlos.

### Formulierungsbeispiele:

### Beispiel 25:

### Sonnenschutzcreme mit Insektenschutz

| Sonnenschutzcreme mit Insektenschutz | |
|---|---|
| | Gew.-Tle. |
| Ceteareth-6 (und) Stearylalkohol | 1,00 |
| Ceteareth-25 | 1,00 |
| Glycerylstearat SE | 6,00 |
| Cetearylalkohol | 0,50 |
| Isopropylpalmitat | 6,00 |
| Octylmethoxycinnamat | 3,00 |
| Dimethylphthalat | 5,00 |
| Benzophenon-3 | 2,00 |
| Polymer aus Beispiel 9 | 5,00 |
| Carbomer | 0,30 |
| Dinatrium-EDTA | 0,05 |
| Propylenglykol | 6,00 |
| Konservierungsmittel | q.s. |
| Wasser | 60,60 |
| Tetrahydroxypropylethylendiamin | 0,55 |
| PEG-25-p-Aminobenzoesäure | 3,00 |
| Parfümöl | q.s. |

### Beispiel 26:

### Sonnenschutzmilch, Typ W/O

| Sonnenschutzmilch, Typ W/O | |
|---|---|
| | Gew.-Tle. |
| PEG-7 hydriertes Castoröl | 6,00 |
| PEG-40 hydriertes Castoröl | 0,50 |
| PEG-45/Dodecylglykol-Copolymer | 2,00 |
| Isopropylmyristat | 8,00 |
| Paraffinöl dickflüssig | 5,00 |
| Jojobaöl | 6,00 |
| Octylmethoxycinnamat | 7,00 |
| Benzophenon-3 | 2,00 |
| Magnesiumstearat | 0,80 |
| Polymer aus Beispiel 7 | 2,00 |
| Konservierungsmittel | q.s. |
| Magnesiumsulfat-7H₂0 | 0,50 |
| Glycerin 87%ig | 5,00 |
| Dinatrium EDTA | 0,10 |
| PEG-25-p-Aminobenzoesäure | 5,00 |
| Wasser | 50,1 |
| Parfümöl | q.s. |

### Beispiel 27:

### Sonnenschutzlotion, Typ O/W

| Sonnenschutzlotion, Typ O/W | |
|---|---|
| | Gew.-Tle. |
| Ceteareth-6 (und) Stearylalkohol | 1,50 |
| Ceteareth-25 | 3,00 |
| Cetearyloctanoat | 3,00 |
| Glycerylstearat | 1,00 |
| Cetearylalkohol | 3,00 |
| Dimethicon | 0,50 |
| Octylsalicylat | 5,00 |
| Benzophenon-3 | 6,00 |
| Octocrylen | 10,00 |
| Polymer aus Beispiel 7 | 2,00 |
| 1,2-Propylenglykol | 5,00 |
| Konservierungsmittel | q.s. |
| Carbomer | 0,25 |
| Dinatrium-EDTA | 0,20 |
| Wasser | 59,20 |
| Tetrahydroxypropylethylendiamin | 0,35 |
| Parfümöl | q.s. |

### Beispiel 28:

### Babycreme

| Babycreme | |
|---|---|
| | Gew.-Tle. |
| PEG-7 hydriertes Castoröl | 6,00 |
| Vaseline | 10,00 |
| Bienenwachs | 2,00 |
| Eucerinum anhydricum | 1,00 |
| Mikrokristallines Wachs | 2,00 |
| Aluminiumstearat | 0,50 |
| Magnesiumstearat | 0,50 |
| Cetearyloctanoat | 8,00 |
| (-)-α-Bisabol nat. | 0,20 |
| Polymer aus Beispiel 11 | 10,00 |
| D-Panthenol USP | 8,00 |
| 1,2-Propylenglykol USP | 3,00 |
| Zinkoxid | 10,00 |
| Konservierungsmittel | q.s. |
| Wasser dest. | 38,8 |
| Parfümöl | q.s. |

### Beispiel 29:

### Körpermilch, Typ O/W

| Körpermilch, Typ O/W | |
|---|---|
| | Gew.-Tle. |
| Ceteareth-6 (und) Stearylalkohol | 1,50 |
| Ceteareth-25 | 1,50 |
| Glycerylstearat | 3,00 |
| Cetylalkohol | 0,20 |
| Cetearyloctanoat | 5,00 |
| Paraffinöl, dickflüssig | 5,00 |
| Lanolin | 2,50 |
| (-)-α-Bisabolol nat. | 0,10 |
| Polymer aus Beispiel 3 | 3,00 |
| 1,2-Propylenglykol USP | 3,00 |
| Panthenol | 2,00 |
| Carbomer | 0,15 |
| Konservierungsmittel | q.s. |
| Wasser | 72,85 |
| Tetrahydroxypropylethylendiamin | 0,20 |
| Parfümöl | q.s. |

### Beispiel 30:

### Gesichtsmaske

| Gesichtsmaske | |
|---|---|
| | Gew.-Tle. |
| Ceteareth-6 (und) Stearylalkohol | 3,00 |
| Ceteareth-25 | 1,50 |
| Cetearylalkohol | 5,00 |
| Cetearyloctanoat | 6,00 |
| Paraffinöl, dickflüssig | 6,00 |
| (-)-α-Bisabolol nat. | 0,20 |
| Glycerinmonostearat | 3,00 |
| Polymer aus Beispiel 8 | 8,00 |
| 1,2-Propylenglykol USP | 2,00 |
| D-Panthenol USP | 5,00 |
| Konservierungsmittel | q.s. |
| Wasser | 59,8 |
| Parfümöl | q.s. |
| Tocopherylacetat | 0,50 |

### Beispiel 31:

### Handcreme

| Handcreme | |
|---|---|
| | Gew.-Tle. |
| Ceteareth-6 (und) Stearylalkohol | 2,00 |
| Ceteareth-25 | 2,00 |
| Cetearyloctanoat | 4,00 |
| Cetearylalkohol | 3,50 |
| Glycerylstearat SE | 3,50 |
| Paraffinöl, dickflüssig | 10,00 |
| (-)-α-Bisabolol nat. | 0,50 |
| Polymer aus Beispiel 11 | 6,00 |
| Panthenol | 4,00 |
| 1,2-Propylenglykol USP | 3,00 |
| Konservierungsmittel | q.s. |
| Wasser | 59,50 |
| Parfümöl | q.s. |
| Sojabohnenextrakt | 2,00 |

### Beispiel 32:

### Make Up

| Make Up | |
|---|---|
| | Gew.-Tle. |
| Glycerinmonostearat | 1,80 |
| Cetylalkohol | 1,80 |
| Ceteareth-6 (und) Stearylalkohol | 1,80 |
| Ceteareth-25 | 1,80 |
| Caprylsäure-/Caprinsäuretriglycerid | 5,30 |
| Paraffinöl, dickflüssig | 5,30 |
| Polymer aus Beispiel 15 | 4,00 |
| 1,2-Proplyenglykol | 4,40 |
| Wasser | 61,50 |
| Konservierungsmittel | q.s. |
| Parfümöl | q.s. |
| Braune Eisenoxide | 1,80 |
| Titandioxid | 10,50 |

### Beispiel 33:

### Lipgloss

| Lipgloss | |
|---|---|
| | Gew.-Tle. |
| Candelillawachs | 5,30 |
| Bienenwachs | 1,10 |
| Mikrokristallines Wachs | 1,10 |
| Cetylpalmitat | 2,00 |
| Paraffinöl, dickflüssig | 3,30 |
| Castoröl (und) Glycerylricincleat (und) Octyldodecanol (und) Carnauba- (und) Candelillawachs | 2,40 |
| (-)-α-Bisabolol nat. | 0,20 |
| Cetearyloctanoat | 16,00 |
| Hydrierte Cocoglyceride | 2,00 |
| Tocopherylacetat | 0,50 |
| Polymer aus Beispiel 2 | 7,00 |
| Rizinusöl DAB 8 | 54,30 |
| Sicomet Azorubinlack E 122 | 0,80 |
| Mica (und) Titandioxid | 4,00 |

### Beispiel 34:

### Lippenstift

| Lippenstift | |
|---|---|
| | Gew.-Tle. |
| Carnaubawachs | 3,00 |
| Candelillawachs | 3,50 |
| Bienenwachs | 2,00 |
| Mikrokristallines Wachs (Permulgin 4200) | 3,50 |
| Mikrokristallines Wachs (Permulgin 3220) | 3,50 |
| Cetylpalmitat | 1,50 |
| Vaseline | 5,50 |
| Lanolinwachs | 3,50 |
| Lanolin | 2,00 |
| Cetearyloctanoat | 10,00 |
| (-)-α-Bisabolol nat. | 0,20 |
| D,L-α-Tocopherol | 0,50 |
| Vitamin-E-Acetat | 2,00 |
| Hydrierte Cocoglyceride | 3,50 |
| Polymer aus Beispiel 5 | 0,50 |
| Rizinusöl DAB 8 | 48,30 |
| Gelbe Eisenoxide | 0,10 |
| Pigment Red 57:1 | 0,10 |
| Pigment Red 63:1 | 0,60 |
| Eisenoxid (Sicopearl Kupfer 1000) | 3,10 |
| Flamenco Red | 3,10 |

### Beispiel 35:

### Mascara

| Mascara | |
|---|---|
| | Gew.-Tle. |
| Carbomer | 0,50 |
| Wasser | 57,5 |
| PVP | 3,00 |
| Wasser | 15,80 |
| Ethanol | 12,00 |
| Polymer aus Beispiel 16 | 0,50 |
| Konservierungsmittel | q.s. |
| Triethanolamin | 0,70 |
| Schwarze Eisenoxide | 10,00 |

### Beispiel 36:

### Augenbrauenstift

| Augenbrauenstift | |
|---|---|
| | Gew.-Tle. |
| Hydrierte Cocoglyceride | 14,0 |
| Capryl-/Caprin-/Stearinsäuretriglyceride | 8,00 |
| Capryl-/Caprinsäuretriglyceride | 8,00 |
| Bis-diglycerylcaprylat/Caprat/Isostearat/Hydroxystearatadipat | 3,00 |
| Hydriertes Palmöl | 2,00 |
| Bienenwachs | 3,00 |
| Polymer aus Beispiel 13 | 1,00 |
| Talkum | 20,00 |
| Titandioxid, mikronisiert | 18,00 |
| Zinkoxid | 18,00 |
| Schwarze Eisenoxide | 5,00 |
| Parfümöl | q.s. |

### Beispiel 37:

### Lidschatten

| Lidschatten | |
|---|---|
| | Gew.-Tle. |
| Talkum | 19,80 |
| Kartoffelstärke | 9,90 |
| Magnesiumstearat | 5,10 |
| Binder | 15,80 |
| Eisenoxide (Sicopearl Kupfer 1000) | 49,40 |

| Binder: | |
|---|---|
| Vaseline | 20,00 |
| Cetearyloctanoat | 10,00 |
| Dimethicon | 5,00 |
| Mikrokristallines Wachs | 5,00 |
| PEG-7 hydriertes Castoröl | 3,00 |
| Polyglyceryl-2-sesquiisostearat (und) Bienenwachs (und) Mineralöl (und) Magnesiumstearat (und) Aluminiumstearat | 5,00 |
| Polymer aus Beispiel 1 | 1,00 |
| Panthenol | 3,00 |
| Konservierungsmittel | q.s. |
| Wasser | 48,00 |
| Parfümöl | q.s. |

## Patentansprüche

1. Verwendung von Polymerisaten oder Copolymerisaten, die aus
a) 40 bis 100 Gew.-% wenigstens eines C₁-C₃₀-(Meth)acrylsäureesters als Monomer A,
b) 0 bis 30 Gew.-% wenigstens eines radikalisch copolymerisierbaren wasserlöslichen Monomers als Monomer B,
c) 0 bis 40 Gew.-% wenigstens eines radikalisch copolymerisierbaren gegebenenfalls N-C₁-C₁₈-Alkyl- oder Hydroxyalkylsubstituierten (Meth)acrylamids als Monomer C und
d) 0 bis 30 Gew.-% wenigstens eines radikalisch copolymerisierbaren Monomers als Monomer D
aufgebaut sind und die durch radikalische Emulsions- oder Suspensionspolymerisation in Gegenwart von wenigstens einem Kettenübertragungsreagenz erhältlich sind, eine Glasübergangstemperatur höher als -35°C und einen Gehalt an organischen flüchtigen Bestandteilen ≤ 0,5 Gew.-% aufweisen in kosmetischen oder pharmazeutischen Mitteln zur Erhöhung der Nassabriebfestigkeit.

2. Verwendung nach Anspruch 1, **dadurch gekennzeichnet, daß** die Menge an Monomer B 1 bis 30 Gew.-% beträgt.

3. Verwendung nach Anspruch 1 oder 2, **dadurch gekennzeichnet, daß** die Menge an Monomer C 1 bis 40 Gew.-% beträgt.

4. Verwendung nach einem der Ansprüche 1 bis 3, **dadurch gekennzeichnet, daß** die Menge an Monomer D 1 bis 30 Gew.-% beträgt.

5. Verwendung nach einem der vorhergehenden Ansprüche, **dadurch gekennzeichnet, daß** das Monomer A ausgewählt ist unter C₂-C₆-(Meth)acrylsäureestern und C₁₂-C₂₂-(Meth)acrylsäureestern.

6. Verwendung nach einem der vorhergehenden Ansprüche, **dadurch gekennzeichnet, daß** das Monomer A ausgewählt ist unter t-Butylacrylat, t-Butylmethacrylat, iso-Butylacrylat oder iso-Butylmethacrylat.

7. Verwendung nach einem der vorhergehenden Ansprüche, **dadurch gekennzeichnet, daß** das Monomer B ausgewählt ist unter monoethylenisch ungesättigten C₃-C₅-Carbonsäuren, insbesondere Acrylsäure und Methacrylsäure, N-Vinylpyrrolidon, N-Vinylvalerolactam, N-Vinylcaprolactam und Hydroxylgruppen-haltigen C₂-C₆-(Meth)acrylsäureestern, insbesondere Hydroxyethyl(meth) acrylat.

8. Verwendung nach einem der vorhergehenden Ansprüche, **dadurch gekennzeichnet, daß** das Monomer C ausgewählt ist unter gegebenenfalls N-C₄-C₁₈-Alkylsubstituierten (Meth)acrylamiden, insbesondere N-t-Butylacrylamid.

9. Verwendung nach einem der vorhergehenden Ansprüche, **dadurch gekennzeichnet, daß** das Monomer D ausgewählt ist unter C₁-C₃₀-Vinylestern, insbesondere C₁₂-C₂₂-Vinylestern, C₁-C₃₀-Vinylethern, insbesondere C₁₂-C₂₂-Vinylethern, vinylaromatischen Verbindungen, insbesondere Styrol und substituierten Styrolen und Si-haltigen Monomeren, insbesondere den Monomeren der Formel I: 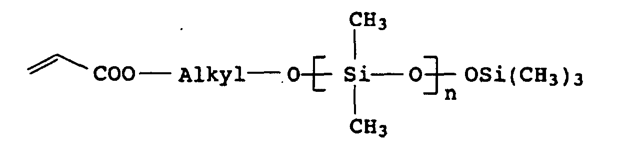 worin n = 6-50 und Alkyl für einen geradkettigen oder verzweigten C₂-C₁₈-Alkylenrest steht.

10. Verwendung nach einem der vorhergehenden Ansprüche, **dadurch gekennzeichnet, daß** das Polymerisat oder Copolymerisat eine Glasübergangstemperatur > 0°C, insbesondere > 35°C, aufweist.

11. Verwendung nach einem der vorhergehenden Ansprüche, **dadurch gekennzeichnet, daß** das Kettenübertragungsreagenz ausgewählt ist unter Aldehyden, SH-Gruppen aufweisenden organischen Verbindungen und halogenierten Kohlenwasserstoffen.

12. Verwendung nach einem der vorhergehenden Ansprüche, **dadurch gekennzeichnet, daß** die Menge an Kettenübertragungsreagenz 0,1 bis 10, insbesondere 0,1 bis 5 Gew.-%, bezogen auf die Menge an Monomer, beträgt.

13. Verwendung nach einem der vorhergehenden Ansprüche, **dadurch gekennzeichnet, daß** das Polymerisat oder Copolymerisat im wesentlichen frei von niedermolekularen Verunreinigungen ist.

14. Verwendung nach einem der vorhergehenden Ansprüche, **dadurch gekennzeichnet, daß** es 0,2 bis 20 Gew.-%, insbesondere 0,5 bis 10 Gew.-%, bezogen auf das Gesamtgewicht des Mittels, des Polymerisates oder Copolymerisates und gegebenenfalls wenigstens einen üblichen Hilfsstoff enthält.

15. Verwendung nach einem der vorhergehenden Ansprüche, **dadurch gekennzeichnet, daß** es in Form einer Creme, Lotion, Milch, eines Öls oder Gels vorliegt.

## Claims

1. The use of polymers or copolymers which are composed of
a) 40-100% by weight of at least one C₁-C₃₀ (meth)acrylic ester as monomer A,
b) 0-30% by weight of at least one water-soluble monomer capable of free-radical copolymerization as monomer B,
c) 0-40% by weight of at least one (meth)acrylamide which is capable of free-radical copolymerization and is unsubstituted or N-substituted by C₁-C₁₈-alkyl or hydroxyalkyl as monomer C and
d) 0-30% by weight of at least one monomer capable of free-radical copolymerization as monomer D,
which are obtainable by free-radical emulsion or suspension polymerization in the presence of at least one chain-transfer reagent, have a glass transition temperature above -35°C and a content of organic volatile constituents ≤ 0.5% by weight, in cosmetic or pharmaceutical composition for increasing the wet substantivity.

2. The use as claimed in claim 1, wherein the amount of monomer B is 1-30% by weight.

3. The use as claimed in claim 1 or 2, wherein the amount of monomer C is 1-40% by weight.

4. The use as claimed in any of claims 1 to 3, wherein the amount of monomer D is 1-30% by weight.

5. The use as claimed in any of the preceding claims, wherein monomer A is selected from C₂-C₆ (meth)acrylic esters and C₁₂-C₂₂ (meth)acrylic esters.

6. The use as claimed in any of the preceding claims, wherein monomer A is selected from t-butyl acrylate, t-butyl methacrylate, isobutyl acrylate or isobutyl methacrylate.

7. The use as claimed in any of the preceding claims, wherein monomer B is selected from monoethylenically unsaturated C₃-C₅ carboxylic acids, in particular acrylic acid and methacrylic acid, N-vinylpyrrolidone, N-vinylvalerolactam, N-vinylcaprolactam and hydroxyl-containing C₂-C₆ (meth)acrylic esters, especially hydroxyethyl (meth)acrylate.

8. The use as claimed in any of the preceding claims, wherein monomer C is selected from (meth)acrylamides which are unsubstituted or N-substituted by C₄-C₁₈-alkyl, in particular N-t-butylacrylamide.

9. The use as claimed in any of the preceding claims, wherein monomer D is selected from C₁-C₃₀ vinyl esters, in particular C₁₂-C₂₂ vinyl esters, C₁-C₃₀ vinyl ethers, especially C₁₂-C₂₂ vinyl ethers, vinyl aromatic compounds, in particular styrene and substituted styrenes and Si-containing monomers, in particular the monomers of formula I: 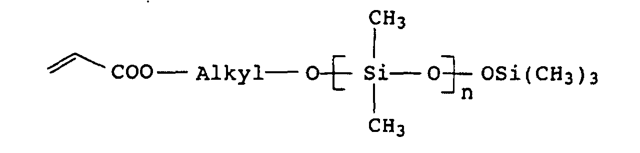 where n is 6-50 and alkyl is a straight-chain or branched C₂-C₁₈-alkylene radical.

10. The use as claimed in any of the preceding claims, wherein the polymer or copolymer has a glass transition temperature > 0°C, in particular > 35°C.

11. The use as claimed in any of the preceding claims, wherein the chain-transfer reagent is selected from aldehydes, SH-containing organic compounds and halogenated hydrocarbons.

12. The use as claimed in any of the preceding claims, wherein the amount of chain-transfer reagent is 0.1-10, in particular 0.1-5, % of the weight of monomer.

13. The use as claimed in any of the preceding claims, wherein the polymer or copolymer is essentially free of low molecular weight impurities.

14. The use as claimed in any of the preceding claims, which comprises 0.2-20% by weight, in particular 0.5-10% by weight, based on the total weight of the composition, of the polymer or copolymer and, where appropriate, at least one conventional ancillary substance.

15. The use as claimed in any of the preceding claims, which is in the form of a cream, lotion, milk, oil or gel.

## Revendications

1. Utilisation de polymères ou copolymères constitués de :
a) 40 à 100% en poids d'au moins un ester (méth)acrylique en C1-C30, en tant que monomère A,
b) 0 à 30% en poids d'au moins un monomère hydrosoluble apte à la copolymérisation radicalaire, en tant que monomère B,
c) 0 à 40% en poids d'un (méth)acrylamide éventuellement substitué par un groupe alkyle ou hydroxyalkyle en C1-C18 à l'azote, apte à la copolymérisation radicalaire, en tant que monomère C et
d) 0 à 30% en poids d'au moins un monomère apte à la copolymérisation radicalaire, en tant que monomère D,
et qu'on peut obtenir par polymérisation radicalaire en émulsion ou en suspension en présence d'au moins un réactif de transfert des chaînes, avec une température de transition du second ordre supérieure à -35°C et une teneur en constituants volatils organiques inférieure ou égale à 0,5% en poids, dans des produits cosmétiques ou pharmaceutiques en vue d'accroître leur résistance à l'abrasion au mouillé.

2. Utilisation selon la revendication 1, **caractérisée en ce que** la proportion du monomère B est de 1 à 30% en poids.

3. Utilisation selon la revendication 1 ou 2, **caractérisée en ce que** la proportion du monomère C est de 1 à 40% en poids.

4. Utilisation selon l'une des revendications 1 à 3, **caractérisée en ce que** la proportion du monomère D est de 1 à 30% en poids.

5. Utilisation selon l'une des revendications qui précèdent, **caractérisée en ce que** le monomère A est choisi parmi les esters (méth)acryliques en C2-C6 et les esters (méth)acryliques en C12-C22.

6. Utilisation selon l'une des revendications qui précèdent, **caractérisée en ce que** le monomère A est choisi parmi l'acrylate de tert-butyle, le méthacrylate de tert-butyle, l'acrylate d'isobutyle et le méthacrylate d'isobutyle.

7. Utilisation selon l'une des revendications qui précèdent, **caractérisée en ce que** le monomère B est choisi parmi les acides carboxyliques à insaturation mono-éthylénique en C3-C5, plus spécialement parmi l'acide acrylique et l'acide méthacrylique, la N-vinylpyrrolidone, le N-vinylvalérolactame, le N-vinylcaprolactame et les esters (méth)acryliques en C2-C6 à groupes hydroxy, en particulier le (méth)acrylate d'hydroxyéthyle.

8. Utilisation selon l'une des revendications qui précèdent, **caractérisée en ce que** le monomère C est choisi parmi les (méth)acrylamides éventuellement substitués à l'azote par un groupe alkyle en C4-C 18, en particulier le N-tert-butylacrylamide.

9. Utilisation selon l'une des revendications qui précèdent, **caractérisée en ce que** le monomère D est choisi parmi les esters vinyliques en C1-C30, plus spécialement les esters vinyliques en C12-C22, les éthers vinyliques en C1-C30, plus spécialement les éthers vinyliques en C12-C22, les composés vinylaromatiques, en particulier le styrène et les styrènes substitués et des monomères contenant du silicium, en particulier les monomères de formule I : 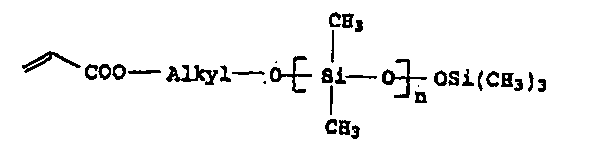 dans laquelle n est un nombre allant de 6 à 50 et « Alkyl » représente un groupe alkylène à chaîne droite ou ramifiée en C2-C18.

10. Utilisation selon l'une des revendications qui précèdent, **caractérisée en ce que** le polymère ou copolymère a une température de transition du second ordre supérieure à 0°C et plus particulièrement supérieure à 35°C.

11. Utilisation selon l'une des revendications qui précèdent, **caractérisée en ce que** le réactif de transfert des chaînes est choisi parmi les aldéhydes, les composés organiques à groupes SH et les hydrocarbures halogénés.

12. Utilisation selon l'une des revendications qui précèdent, **caractérisée en ce que** la quantité du réactif de transfert des chaînes représente de 0,1 à 10%, plus spécialement de 0,1 à 5% du poids des monomères.

13. Utilisation selon l'une des revendications qui précèdent, **caractérisée en ce que** le polymère ou copolymère est essentiellement exempt d'impuretés à bas poids moléculaire.

14. Utilisation selon l'une des revendications qui précèdent, **caractérisée en ce que** le produit contient 0,2 à 20%, plus spécialement 0,5 à 10% de son poids total du polymère ou copolymère, et le cas échéant au moins un produit auxiliaire usuel.

15. Utilisation selon l'une des revendications qui précèdent, **caractérisée en ce que** le produit est sous forme de crème, de lotion, de lait, d'huile ou de gel.
